(19) Europäisches Patentamt
European Patent Office
Office européen des brevets

(11) EP 3 241 564 A1

(12) EUROPEAN PATENT APPLICATION
published in accordance with Art. 153(4) EPC

(43) Date of publication:
08.11.2017 Bulletin 2017/45

(51) Int Cl.:
A61K 47/38 (2006.01)     A61K 47/10 (2017.01)
A61K 9/20 (2006.01)     A61K 9/48 (2006.01)

(21) Application number: 15875446.5

(22) Date of filing: 08.05.2015

(86) International application number:
PCT/KR2015/004625

(87) International publication number:
WO 2016/108351 (07.07.2016 Gazette 2016/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 31.12.2014 KR 20140195965

(71) Applicant: LOTTE Fine Chemical Co., Ltd.
Ulsan, 44714 (KR)

(72) Inventors:
• CHA, Jae Uk
  Incheon 406-818 (KR)
• LEE, Sung Wan
  Incheon 402-803 (KR)

(74) Representative: Kador & Partner
Corneliusstraße 15
80469 München (DE)

(54) COMPOSITION FOR FORMING COMPLEX, COMPLEX FORMED THEREFROM, AND COMPOSITION FOR ORAL INGESTION, CONTAINING SAME

(57) Disclosed are: a composition for forming a complex; a complex formed therefrom; and a composition for oral ingestion, containing the same. The disclosed composition for forming a complex contains a cellulose-based compound, a polyphenolic compound, a gelling agent, and a solvent.

EP 3 241 564 A1

**Description**

TECHNICAL FIELD

The inventive concept relates to a composition for forming a complex, a complex formed therefrom, and an orally ingestible composition including the complex, and more particularly, to a composition for forming a complex having pH-dependent dissolution characteristics, a composite formed from the complex, and an orally ingestible composition including the complex.

BACKGROUND ART

In the pharmaceutical or food field, film is used to physically and chemically protect active main components. A film in the pharmaceutical field is used to improve the quality of pharmaceutical products by protecting them from the environment, such as moisture, oxygen, or light and shielding the taste, smell, or color of active main components. Especially, a film using a compound having pH-dependent dissolution characteristics is also referred to as enteric film.
When the enteric film is used in the pharmaceutical field, a compound, such as polymethacrylate, hydroxypropyl methylcellulose phthalate, polyvinylacrylate phthalate, cellulose acetate phthalate, or hydroxypropylmethylcellulose acetate succinate may be used as a raw material of the enteric film.
When the enteric film is used in food, a raw material of the enteric film is limited to alginates or shellac. The enteric film is, for example, prepared by coating a tablet with a composition for forming enteric film, and drying the coating composition.
However, conventional enteric film cannot be used in some food materials, alginates or shellac that may be used in food materials does not have sufficient pH-dependent dissolution characteristics, thus dissolves slowly not only in the stomach but also in the intestine, not providing a satisfactory enteric property that a user desires. Also, the pharmaceutical products have low transparency, easily breakable under a condition of low moisture concentration, and have sticky characteristics, and thus an additional excipient such as a plasticizer or talc needs to be used in a large amount. Also, a material containing phthalate has a problem of free phthalic acid dissociation.

DETAILED DESCRIPTION OF THE INVENTIVE CONCEPT

TECHNICAL PROBLEM

The inventive concept provides a composition for forming a complex having pH dependent dissolution characteristics.
The inventive concept provides a complex formed from the composition for forming a complex.
The inventive concept provides an orally ingestible composition including the complex.

TECHNICAL SOLUTION

According to an aspect of the inventive concept, there is provided a composition for forming a complex, the composition including a cellulose-based compound; a polyphenolic compound; a gelling agent; and a solvent, wherein an amount of the polyphenolic compound is in a range of 1 part to 40 parts by weight based on 100 parts by weight of the cellulose-based compound.
The cellulose-based compound may include hydroxypropylmethylcellulose (HPMC), methylcellulose (MC), carboxymethylcellulose (CMC), derivatives thereof, or combinations thereof.
The polyphenolic compound may include catechin, chrysin, hesperetin, hesperidin, quercetin, baicalin, flavone, naringenin, naringin, resveratrol, or combinations thereof.
An amount of the polyphenolic compound may be in a range of 1 part to 40 parts by weight based on 100 parts by weight of the cellulose-based compound.
The gelling agent may include gellan gum, xanthan gum, sodium alginate, gelatin, alginic acid, sodium carboxymethylcellulose, poloxamer, polyvinyl alcohol, or combinations thereof.
An amount of the gelling agent may be in a range of 5 parts to 15 parts by weight based on 100 parts by weight of the cellulose-based compound.
The solvent may include at least one selected from water and ethanol.
An amount of the solvent may be in a range of 100 parts to 2,000 parts by weight based on 100 parts by weight of the total weight of all components other than the solvent.
The composition for forming a complex may further include an antioxidant at an amount in a range of 1 part to 10 parts by weight based on 100 parts by weight of the cellulose-based compound.

The antioxidant may include ascorbic acid, butylhydroxyanisole, erythorbic acid, propyl gallate, rosemary oil, dibutyl hydroxy toluene, tocopherol, or combinations thereof.

The composition for forming a complex may further include a pH controlling agent.

The pH controlling agent may include an alkaline agent selected from sodium hydroxide, potassium hydroxide, calcium hydroxide, or combinations thereof.

According to another aspect of the inventive concept, there is provided a complex including a cellulose-based compound; a polyphenolic compound; and a gelling agent.

The complex may have pH-dependent dissolution characteristics.

The complex may have a controllable soluble pH range of 3 to 7.

According to another aspect of the inventive concept, there is provided a composition for oral ingestion including the complex.

ADVANTAGEOUS EFFECTS

Unlike conventional chemical materials, the complex according to an exemplary embodiment of the present invention has no toxicity, harmful substances are not generated when the complex is decomposed, and the complex may be used in all industrial fields as a food material. Also, when used in the human body, the complex may ultimately provide a physiological function such as antioxidation, and since the complex has an antioxidant ingredient, such as green tea, the complex may prevent oxidation of a material and thus may protect a component covered with a film prepared by using the complex. In terms of process, a process for producing the complex does not require additional chemical energy due to simple dissolving and washing after reaction, and thus the process is very simple, environment-friendly, and inexpensive. Also, the complex is dissolved in an organic solvent (e.g., ethanol) and thus may easily form a film, and the film thus formed has excellent characteristics. Moreover, the complex includes a gelling agent in addition to a polyphenolic compound and a cellulose-based compound, which are natural products, and thus a soluble pH range that is controllable may be broadened from a conventional pH range of 5 to 6 to a pH range of 3 to 7. Therefore, when a soluble pH of the film prepared by using the complex is appropriately controlled, the film may be controlled to be disintegrated at a desired site in a small intestine including duodenum, jejunum, or ileum.

BEST MODE

As used herein, a degree of substitution of a methoxyl group, a degree of substitution of a hydroxypropoxyl group, and degrees of substitution of other substituents of hydroxypropylmethyl cellulose may be calculated according to Equation 1:

Equation 1

Degree of substitution of particular substituent (wt%) = Total weight of particular substituent / total weight of hydroxypropylmethylcellulose × 100

Hereinafter, a composition for forming a complex according to an embodiment of the present invention will be described in detail.

The composition for forming a complex according to an embodiment of the present invention includes a cellulose-based compound, a polyphenolic compound, a gelling agent, and a solvent.

The cellulose-based compound forms a film that performs an enteric function by wrapping a tablet or a capsule.

The cellulose-based compound may include hydroxypropylmethylcellulose (HPMC), methylcellulose (MC), carboxy methylcellulose (CMC), derivatives thereof, or combinations thereof.

The HPMC may have a degree of substitution of a methoxyl group in a range of 16.5 to 30.0 wt% and a degree of substitution of a hydroxypropoxyl group in a range of 4 to 32 wt%. When an amount of the methoxyl group and an amount of the hydroxypropoxyl group in the HPMC are within these ranges, each respectively, a hydrophobic group and a hydrophilic group are appropriately balanced, which is preferable in formation of a complex, and a film-forming property is excellent after the complex formation. Therefore, excellent film quality may be secured when an enteric film is prepared by using the HPMC.

For example, the HPMC may have a degree of substitution of a methoxyl group in a range of 28 to 30 wt% and a degree of substitution of a hydroxypropoxyl group in a range of 7 to 12 wt%.

The polyphenolic compound may give a film-forming property to the composition for forming a complex and may give pH dependent dissolution characteristics and toughness to the complex and film formed by using the composition for forming a complex. As used herein, the term "pH dependent dissolution characteristics" refers to a property of a material that is dissolved in a particular pH range but is not dissolved in pH ranges other than the particular pH range.

The polyphenolic compound may be obtained from an aerial part of a natural material. For example, the polyphenolic compound may be obtained from various teas or fruits. For example, the polyphenolic compound may be obtained from a green tea extract.

Polyphenols constitute most part of the green tea extract other than a water part thereof. Polyphenols included in the green tea extract may include catechins such as epigallocatechin gallate (EGCG), epicatechin gallate (ECG), epicatechin (EC), and epigallocatechin (EGC).

Also, the polyphenolic compound may include crysin, hesperetin, hesperidin, quercetin, baicalin, flavone, naringenin, naringin, resveratrol, or a combination thereof.

The green tea extract may be prepared according to a method commonly used in the art. For example, the green tea extract may be prepared by undergoing a process of extracting green tea by using a mixed solvent of ethanol and water, separating the green tea extract from the resultant, and drying the green tea extract. The green tea extract thus prepared may be in the form of paste or a powder.

As a result, the polyphenolic compound may include catechins, crysin, hesperetin, hesperidin, quercetin, baicalin, flavone, naringenin, naringin, resveratrol, or a combination thereof.

An amount of the polyphenolic compound may be in a range of 1 part to 40 parts by weight based on 100 parts by weight of the cellulose-based compound. When the amount of the polyphenolic compound is within this range, a complex formed from the composition for forming a complex may exhibit pH dependent dissolution characteristics, and when a film is prepared by using the complex formed from the composition for forming a complex, the film having excellent film-forming property and excellent toughness may be obtained.

An amount of the polyphenolic compound may be, for example, in a range of 5 parts to 30 parts by weight based on 100 parts by weight of the cellulose-based compound.

The gelling agent may broaden controllable soluble pH ranges of the complex and film formed from the composition for forming a complex.

The gelling agent may include gellan gum, xanthan gum, sodium alginate, gelatin, alginic acid, sodium carboxymethylcellulose, poloxamer, polyvinyl alcohol, or a combination thereof.

An amount of the gelling agent may be in a range of 5 parts to 15 parts by weight based on 100 parts by weight of the cellulose-based compound.

When the amount of the gelling agent is within this range, a film sufficiently exhibiting an enteric function and having excellent physical properties may be formed.

The solvent may include at least one selected from water and ethanol.

An amount of the solvent may be in a range of 100 parts to 2,000 parts by weight based on 100 parts by weight of the total weight of all components other than the solvent. When the amount of the solvent is within this range, components that constitute the composition for forming a complex may be homogenously mixed, and thus a yield of the complex may increase when the complex is formed by using the composition for forming a complex.

The composition for forming a complex may further include an antioxidant.

The antioxidant may suppress long-term storage stability decrease occurred by interaction between the cellulose-based compound and the polyphenolic compound.

The antioxidant may include ascorbic acid, butylhydroxyanisole, erythorbic acid, propyl gallate, rosemary oil, dibutyl hydroxy toluene, tocopherol, or a combination thereof.

An amount of the antioxidant may be in a range of 1 part to 10 parts by weight based on 100 parts by weight of the cellulose-based compound. When the amount of the antioxidant is within this range, a complex and a film having excellent long-term storage stability improving effect and an enteric function may be provided.

The composition for forming a complex may further include a pH controlling agent.

The pH controlling agent increases a solubility of a solid material in the composition for forming a complex.

The pH controlling agent may include an alkaline agent.

The alkaline agent may include sodium hydroxide, potassium hydroxide, calcium hydroxide, or a combination thereof.

An amount of the pH controlling agent may be in a range of greater than 0 to 10 parts by weight based on 100 parts by weight of the cellulose-based compound. When the amount of the pH controlling agent is within this range, a turbidity and a strength of a film including the complex prepared by using the composition for forming a complex may improve. An amount of the pH controlling agent may be, for example, in a range of 0.01 part to 7 parts by weight based on 100 parts by weight of the cellulose-based compound.

According to another aspect of an embodiment of the present invention, provided is a complex including the cellulose-based compound, the polyphenolic compound and the gelling agent.

The complex may further include at least one selected from the antioxidant and the pH controlling agent.

The complex may have pH dependent dissolution characteristics.

The complex may have a controllable soluble pH range of 3 to 7. As used herein, the term "the complex having a controllable soluble pH range of 3 to 7" denotes that a pH of a solvent in which the complex may be dissolved, that is, a soluble pH, may be freely controlled to an arbitrary value between 3 to 7 (e.g., 3, 4, 5, 6, or 7).

Despite including only safe food materials, the complex has pH dependent dissolution characteristics and thus may have enteric property of the same level with those of an enteric film formed of a pharmaceutical compound. Further, the complex may exhibit beneficial effects on health along with antioxidizing effects provided by the polyphenolic compound, may overcome disadvantageous characteristics of the pharmaceutical material, and thus may have excellent qualities.

The complex may be used in a general film as well as in an enteric film and may be used in all areas that require pH dependent dissolution characteristics.

The complex has a characteristic of being dissolved in a solvent, such as water, in various pH ranges according to a mixing ratio of the cellulose-based compound, the polyphenolic compound, and the gelling agent. Also, when the complex is dissolved in some organic solvents, for example, a mixed solvent of ethanol and water at a weight ratio of 8:2, the complex may have excellent film-forming property, and the film ultimately formed may have pH dependent dissolution characteristics. Therefore, when the complex is used, a film may be easily prepared, and the film may be useful in fields including the pharmaceutical and food areas where a pH dependent dissolution characteristic is needed.

When the complex is used as an enteric film or a material for a capsule, the composition for forming a complex may further include additives such as plasticizers, lubricants, colorants, light-blocking agents, and solubilizers.

The complex may have a soluble pH range that changes according to a composition of the complex. Therefore, the complex may be used in various areas such as a binder or a filler of a tablet, coating of a capsule or a tablet, and a base material of an enteric hard or soft capsule.

Also, since the complex includes a vegetable material such as a green tea extract, it is non-toxic and safe, and can be used in various areas such as the food and pharmaceutical products. Moreover, a manufacturing process of the complex is very simple, which is advantageous in terms of low manufacturing cost. Also, since the complex includes a polyphenolic compound, it is excellent in physiological functions such as antioxidative action and reduction of body fat, and has a function of preventing oxidation of a substance protected by the complex.

Hereinafter, the composition for forming a complex and a method of preparing a complex will be described in detail.

The first step is dissolving all components other than the solvent (e.g., the cellulose-based compound, the polyphenolic compound, the gelling agent, and, optionally, at least one selected from the antioxidant and the pH controlling agent) in the solvent to obtain a composition for forming a complex.

The first step may include a process of dissolving all components other than the solvent in the solvent at once by adding all the components other than the solvent to the solvent at the same time.

The first step may include a process of obtaining a plurality of solutions by dissolving each of all the components other than the solvent in the solvent; and mixing these solutions.

The total amount of the solvent may be in a range of 100 parts to 2,000 parts by weight based on 100 parts by weight of the total weight of all the components other than the solvent. When the total amount of the solvent is within this range, a composition for forming a complex, in which all the components other than the solvent are homogenously dissolved in the solvent, may be obtained.

The second step is stirring the composition for forming a complex to form a complex. In the second step, at least two materials are allowed to react with each other and form a complex. Here, the complex is obtained in the form of a precipitate that is not dissolved in the solvent.

The third step is pouring out the solvent from the mixed solution including the complex and drying the complex to obtain a dried complex. The drying may be performed by evaporation under reduced-pressure, spray drying, or natural-drying. Also, the drying may be performing at a temperature in a range of 15 to 150°C, for example, 50 to 60°C.

The fourth step is washing the dried complex with a washing solution, in which the dried complex is not dissolved, to remove unreacted materials and impurities from the dried complex. As a result, a complex having a high purity may be obtained.

The washing solution may be any liquid having a pH lower than the pH range in which the complex is dissolved. For example, the washing solution may be purified water that does not have pH buffering capacity.

The composition for oral ingestion may be a preparation of a solid state, such as tablets, capsules, granules, or powders, and the composition may be used in food, health functional food, or pharmaceutical products.

An amount of the complex in the composition for oral ingestion may be in a range of 0.5 to 80 wt%. When the amount of the complex is within this range, the composition for oral ingestion having excellent physiological function may be obtained.

The composition for oral ingestion may further include the pH controlling agent.

The composition for oral ingestion may further include the solvent.

An amount of the pH controlling agent and an amount of the solvent may be varied depending on components and use of the composition for oral ingestion.

MODE OF THE INVENTIVE CONCEPT

Hereinafter, the present invention will be described in detail by referring to Examples, but the present invention is not limited to these Examples.

Example

Examples 1 to 21 and Comparative Examples 1 to 13: Preparation of complex and film

(Preparation of complex)

As shown in Table 1, a composition for forming a complex was obtained by mixing hydroxypropylmethylcellulose (HPMC), a polyphenolic compound, a gelling agent, and a 60 wt% ethanol (EtOH) aqueous solution. Then, a liquid component in the composition for forming a complex was removed by using a Rotary evaporator at 50 to 60°C, and thus a dried complex was obtained. Thereafter, 50 g of the complex was added to 500 ml of purified water, and the resultant was stirred for about 5 minutes. The resultant was filtered to obtain a solid, and the solid was dried in an oven at a temperature of about 80°C to obtain a complex having a high-purity from which impurities and unreacted materials are removed.

[Table 1]

| Sample | 60 wt% EtOH (g) | HPMC[*1] (g) | Polyphenolic compound | | Gelling agent | |
|---|---|---|---|---|---|---|
| | | | Type | Amount (g) | Type | Amount (g) |
| Example 1 | 100 | 9 | Crysin | 1 | Gellan gum A[*3] | 0.25 |
| Example 2 | 100 | 9 | Crysin | 1 | Gellan gum B[*4] | 0.25 |
| Example 3 | 100 | 9 | Crysin | 1 | Gellan gum C[*5] | 0.25 |
| Example 4 | 100 | 9 | EGCG[*2] | 1 | Gellan gum A | 0.25 |
| Example 5 | 100 | 9 | EGCG | 1 | Gellan gum B | 0.25 |
| Example 6 | 100 | 9 | EGCG | 1 | Gellan gum C | 0.25 |
| Example 7 | 100 | 9 | Hesperetin | 1 | Gellan gum B | 0.25 |
| Example 8 | 100 | 9 | Hesperetin | 1 | Gellan gum C | 0.25 |
| Example 9 | 100 | 9 | Hesperidin | 1 | Gellan gum A | 0.25 |
| Example 10 | 100 | 9 | Hesperidin | 1 | Gellan gum B | 0.25 |
| Example 11 | 100 | 9 | Hesperidin | 1 | Gellan gum C | 0.25 |
| Example 12 | 100 | 9 | Quercetin | 1 | Gellan gum A | 0.25 |
| Example 13 | 100 | 9 | Quercetin | 1 | Gellan gum B | 0.25 |
| Example 14 | 100 | 9 | Quercetin | 1 | Gellan gum C | 0.25 |
| Example 15 | 100 | 9 | EGCG | 1 | Gellan gum B | 0.10 |
| Example 16 | 100 | 9 | EGCG | 1 | Gellan gum B | 0.25 |
| Example 17 | 100 | 9 | EGCG | 1 | Gellan gum B | 0.40 |
| Example 18 | 100 | 9 | EGCG | 0.75 | Gellan gum B | 0.25 |
| Example 19 | 100 | 9 | EGCG | 1.25 | Gellan gum B | 0.25 |
| Example 20 | 100 | 9 | EGCG | 1 | Xanthan gum | 0.25 |
| Example 21 | 100 | 9 | EGCG | 1 | Sodium alginate | 0.25 |
| Comparative Example 1 | 100 | 9 | - | 0 | - | 0 |
| Comparative Example 2 | 100 | 9 | Baicalin | 1 | - | 0 |
| Comparative Example 3 | 100 | 9 | Crysin | 1 | - | 0 |

(continued)

| Sample | 60 wt% EtOH (g) | HPMC*1 (g) | Polyphenolic compound | | Gelling agent | |
|---|---|---|---|---|---|---|
| | | | Type | Amount (g) | Type | Amount (g) |
| Comparative Example 4 | 100 | 9 | Flavone | 1 | - | 0 |
| Comparative Example 5 | 100 | 9 | Hesperetin | 1 | - | 0 |
| Comparative Example 6 | 100 | 9 | Hesperetin | 1 | - | 0 |
| Comparative Example 7 | 100 | 9 | Naringenin | 1 | - | 0 |
| Comparative Example 8 | 100 | 9 | Naringin | 1 | - | 0 |
| Comparative Example 9 | 100 | 9 | Quercetin | 1 | - | 0 |
| Comparative Example 10 | 100 | 9 | Resveratrol | 1 | - | 0 |
| Comparative Example 11 | 100 | 9 | EGCG | 1 | - | 0 |
| Comparative Example 12 | 100 | 9 | EGCG | 0.75 | - | 0 |
| Comparative Example 13 | 100 | 9 | EGCG | 1.25 | - | 0 |
| *1: HPMC2910 (AnyCoat-C® AN Grade) available from Samsung Fine Chemicals Co., Ltd *2: Epigallocatechin gallate *3: Kelcogel® High acyl (Gellan gum (High acyl)) available from CP Kelco *4: Kelcogel® Low acy 1(Gellan gum (Low acyl)) available from CP Kelco *5: Gellan gum (Agar sub) available from Sigma-Aldrich | | | | | | |

(Preparation of film)

1 g of each of the complexes prepared in Examples 1 to 21 and Comparative Examples 1 to 13 was dissolved in 5 ml of a 80 wt% ethanol aqueous solution to prepare a complex solution. Thereafter, the complex solution was cast on a glass substrate, and the resultant was natural-dried to obtain a film having a thickness of about 60 $\mu$m. As used herein, the term "natural-drying" refers to drying overnight at room temperature (about 25°C).

Evaluation Example: Soluble pH evaluation

Each of the films prepared from Examples 1 to 21 and Comparative Examples 1 to 13 was added to a plurality of Britton-Robinson buffers having various pH ranges, and the resultant was stirred at 37°C for 1 hour to observe whether each of the films was dissolved in each of the Britton-Robinson buffers.
The Britton-Robinson buffers were prepared by adding 2.7 mL of 99.9 wt% phosphoric acid and 2.47 g of boric acid to 2.5 mL of galacial acetic acid, further adding purified water thereto so that the total volume was 1,000 mL, and dropwisely adding a 2 N sodium hydroxide solution to the resultant.
An amount of the dropwisely added 2 N sodium hydroxide solution was controlled to prepare the Britton-Robinson buffers having various pH ranges.
Soluble pH ranges of the films were evaluated, and the results are shown in Table 2.

[Table 2]

| Sample | Soluble pH | Sample | Soluble pH |
|---|---|---|---|
| Example 1 | 4.0 | Example 18 | 5.0 |
| Example 2 | 5.0 | Example 19 | 5.0 |
| Example 3 | 5.0 | Example 20 | 5.0 |
| Example 4 | 7.0 | Example 21 | 7.0 |

(continued)

| Sample | Soluble pH | Sample | Soluble pH |
|---|---|---|---|
| Example 5 | 5.0 | Comparative Example 1 | 1.2 |
| Example 6 | 5.0 | Comparative Example 2 | 1.2 |
| Example 7 | 5.0 | Comparative Example 3 | 1.2 |
| Example 8 | 3.0 | Comparative Example 4 | 10.0 |
| Example 9 | 5.0 | Comparative Example 5 | 1.2 |
| Example 10 | 6.0 | Comparative Example 6 | 1.2 |
| Example 11 | 4.0 | Comparative Example 7 | 1.2 |
| Example 12 | 5.0 | Comparative Example 8 | 1.2 |
| Example 13 | 5.0 | Comparative Example 9 | 1.2 |
| Example 14 | 4.0 | Comparative Example 10 | 9.0 |
| Example 15 | 5.0 | Comparative Example 11 | 6.0 |
| Example 16 | 5.0 | Comparative Example 12 | 6.0 |
| Example 17 | 5.0 | Comparative Example 13 | 6.0 |

Referring to Table 2, it appeared that the films prepared in Examples 1 to 21 had a soluble pH range that may be freely controlled within a range of 3.0 to 7.0, whereas the films prepared in Comparative Examples 1 to 13 had a soluble pH range that is fixed at one value (e.g., 1.2, 6.0, 9.0, or 10.0) between 1.0 to10.0.

While the inventive concept has been particularly shown and described with reference to exemplary embodiments thereof, it will be understood that various changes in form and details may be made therein without departing from the spirit and scope of the following claims.

**Claims**

1.   A composition for forming a complex, the composition comprising:

a cellulose-based compound;
a polyphenolic compound;
a gelling agent; and
a solvent,
wherein an amount of the polyphenolic compound is in a range of 1 part to 40 parts by weight based on 100 parts by weight of the cellulose-based compound.

2.   The composition of claim 1, wherein the cellulose-based compound comprises hydroxypropylmethylcellulose (HPMC), methylcellulose (MC), carboxymethylcellulose (CMC), derivatives thereof, or combinations thereof.

3.   The composition of claim 1, wherein the polyphenolic compound comprises catechin, chrysin, hesperetin, hesperidin, quercetin, baicalin, flavone, naringenin, naringin, resveratrol, or combinations thereof.

4.   The composition of claim 1, wherein the gelling agent comprises gellan gum, xanthan gum, sodium alginate, gelatin, alginic acid, sodium carboxymethylcellulose, poloxamer, polyvinyl alcohol, or combinations thereof.

5.   The composition of claim 1, wherein an amount of the gelling agent is in a range of 5 parts to 15 parts by weight based on 100 parts by weight of the cellulose-based compound.

6.   The composition of claim 1, wherein the solvent comprises at least one selected from water and ethanol, and an amount of the solvent is in a range of 100 parts to 2,000 parts by weight based on 100 parts by weight of the total weight of all components other than the solvent.

**7.** The composition of claim 1 further comprising an antioxidant at an amount in a range of 1 part to 10 parts by weight based on 100 parts by weight of the cellulose-based compound.

**8.** The composition of claim 7, wherein the antioxidant comprises ascorbic acid, butylhydroxyanisole, erythorbic acid, propyl gallate, rosemary oil, dibutyl hydroxy toluene, tocopherol, or combinations thereof.

**9.** The composition of claim 1 further comprising a pH controlling agent, wherein the pH controlling agent comprises an alkaline agent selected from sodium hydroxide, potassium hydroxide, calcium hydroxide, or combinations thereof.

**10.** A complex comprising:

a cellulose-based compound;
a polyphenolic compound; and
a gelling agent,
wherein an amount of the polyphenolic compound is in a range of 1 part to 40 parts by weight based on 100 parts by weight of the cellulose-based compound.

**11.** The complex of claim 10, wherein an amount of the gelling agent is in a range of 5 parts to 15 parts by weight based on 100 parts by weight of the cellulose-based compound.

**12.** The complex of claim 10 further comprising an antioxidant at an amount in a range of 1 part to 10 parts by weight based on 100 parts by weight of the cellulose-based compound.

**13.** The complex of claim 10 having pH-dependent dissolution characteristics.

**14.** The complex of claim 13 having a controllable soluble pH range of 3 to 7.

**15.** A composition for oral ingestion comprising the complex of claim 10.

## INTERNATIONAL SEARCH REPORT

| International application No. |
|---|
| **PCT/KR2015/004625** |

### A. CLASSIFICATION OF SUBJECT MATTER

*A61K 47/38(2006.01)i, A61K 47/10(2006.01)i, A61K 9/20(2006.01)i, A61K 9/48(2006.01)i*

According to International Patent Classification (IPC) or to both national classification and IPC

### B. FIELDS SEARCHED

Minimum documentation searched (classification system followed by classification symbols)

A61K 47/38; A61K 47/44; A23L 1/29; C08K 5/053; A61K 9/20; C08L 1/02; A61K 7/16; A61K 9/70; A61K 9/28; A61K 7/26; A61K 47/10; A61K 9/48

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: cellulose, polyphenol, gelling agent, solvent, composite, oral feeding, pH-dependent

### C. DOCUMENTS CONSIDERED TO BE RELEVANT

| Category* | Citation of document, with indication, where appropriate, of the relevant passages | Relevant to claim No. |
|---|---|---|
| X | KR 10-2014-0075598 A (SAMSUNG FINE CHEMICALS CO., LTD.) 19 June 2014<br>See abstract; paragraphs [0020], [0035], [0037], [0042], [0062]; claims 1-12. | 1-15 |
| X | US 7803402 B2 (KHANDELWAL, Sanjeev et al.) 28 September 2010<br>See abstract; column 4, lines 30-34; claims 1, 3; examples 1-3. | 1-15 |
| A | US 2014-0335153 A1 (CURE PHARMACEUTICAL CORPORATION)<br>13 November 2014<br>See abstract; paragraphs [0143]-[0144], [0153]-[0154]; claims 1, 3, 9. | 1-15 |
| A | CN 104223078 A (TIANJIN JUXING KANGHUA MEDICAL TECHNOLOGY CO., LTD.) 24 December 2014<br>See abstract; paragraphs [0017]-[0030]; claims 1-8. | 1-15 |
| A | WO 01-17494 A1 (THE PROCTER & GAMBLE COMPANY) 15 March 2001<br>See abstract; claims 1, 4-5, 10. | 1-15 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 31 AUGUST 2015 (31.08.2015) | **31 AUGUST 2015 (31.08.2015)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (January 2015)

**INTERNATIONAL SEARCH REPORT**
Information on patent family members

| International application No. |
|---|
| **PCT/KR2015/004625** |

| Patent document cited in search report | Publication date | Patent family member | Publication date |
|---|---|---|---|
| KR 10-2014-0075598 A | 19/06/2014 | WO 2014-092419 A1 | 19/06/2014 |
| US 7803402 B2 | 28/09/2010 | US 2004-0005361 A1 | 08/01/2004 |
| US 2014-0335153 A1 | 13/11/2014 | NONE | |
| CN104223078 A | 24/12/2014 | NONE | |
| WO 01-17494 A1 | 15/03/2001 | AU 6030299 A | 10/04/2001 |
| | | CN 1367663 A | 04/09/2002 |
| | | CN 1373652 A | 09/10/2002 |

Form PCT/ISA/210 (patent family annex) (January 2015)